**Europäisches Patentamt**

(19) **European Patent Office**

**Office européen des brevets**

(11) Numéro de publication: **0 176 452**
**B1**

(12) # FASCICULE DE BREVET EUROPÉEN

(45) Date de publication du fascicule du brevet:
**08.06.88**

(51) Int. Cl.⁴: **F 16 M 13/00**

(21) Numéro de dépôt: **85420152.2**

(22) Date de dépôt: **20.08.85**

(54) Dispositif de support et de réglage en orientation d'organes divers par rapport à un plan de référence.

(30) Priorité: **22.08.84 FR 8413235**

(43) Date de publication de la demande:
**02.04.86 Bulletin 86/14**

(45) Mention de la délivrance du brevet:
**08.06.88 Bulletin 88/23**

(84) Etats contractants désignés:
**BE DE GB IT NL**

(56) Documents cité:
**FR-A-2 156 966**
**US-A-3 212 497**
**US-A-3 762 404**
**US-A-4 068 655**

(73) Titulaire: **INSTITUT NATIONAL DE LA SANTE ET DE LA RECHERCHE MEDICALE (INSERM), 101, rue de Tolbiac, F-75654 Paris Cédex 13 (FR)**

(72) Inventeur: **Cathignol, Dominique, 14, rue du Fort, F-69740 Genas (FR)**
Inventeur: **Chapelon, Jean- Yves, 6, allée M. Achard, F-69100 Villeurbanne (FR)**
Inventeur: **Devonec, Marian, 81, avenue des Balmes, F-01700 Miribel (FR)**

(74) Mandataire: **Ropital- Bonvarlet, Claude, Cabinet BEAU DE LOMENIE 99, Grande rue de la Guillotière, F-69007 Lyon (FR)**

## Description

La présente invention concerne le domaine technique du support et du réglage, selon une orientation prédéterminée, d'un organe ou appareil quelconque par rapport à un plan de référence stable considéré comme fixe.

Pour régler ce problème, la technique antérieure offre de nombreuses solutions à base d'organes mécaniques pouvant être qualifiés de rotules ou joints universels associés à des organes de blocage propres.

Tous ces mécanismes ont l'inconvénient d'être complexes, onéreux, peu sensibles et difficiles à régler compte tenu des opérations successives d'immobilisation selon les différents axes de mobilité.

En outre, tous ces mécanismes ont en plus l'inconvénient de ne pas permettre aisément un réglage dans plusieurs directions, sinon en faisant intervenir des adaptations spécifiques à chacune d'elles, ce qui accroît encore la complexité du mécanisme et les difficultés de réglage.

Parmi l'une des applications préférées, il y a lieu de citer le maintien selon une direction prédéterminée d'une aiguille de biopsie destinée à permettre le prélèvement de cellues d'un organe quelconque d'un patient immobilisé sur une table d'intervention.

Jusqu'à présent, dans un tel domaine d'application, le support d'une telle aiguille est assuré au moyen d'un portique métallique monté par des noix de fixation sur au moins une règle ou barrette faisant partie de la structure de la table d'intervention.

Le portique comprend différents éléments de structure matérialisant au moins deux directions, ces éléments de structure étant reliés à la table par des noix de réglage possédant des organes d'immobilisation propres.

Le réglage selon une orientation déterminée d'une telle aiguille nécessite donc de procéder par étapes successives pour régler chacune des noix afin que l'ensemble des réglages apportés permette de conférer l'orientation recherchée.

Outre les difficultés de procéder à un tel réglage, il faut remarquer que le portique représente, en outre, un organe métallique sujet aux vibrations. Une action intempestive portée directement sur le portique, modifie ainsi de façon notable l'orientation initiale de l'aiguille.

Il existe donc manifestement dans un tel domaine d'application au moins, le besoin de proposer un dispositif de support et de réglage en orientation, éliminant de tels inconvénients et permettant à un praticien d'une technique donnée, de réaliser rapidement, de façon certaine, un réglage d'orientation précis d'un organe quelconque ne faisant intervenir aucun outillage particulier, ni des connaissances techniques propres à un matériel spécifique.

L'objet de l'invention est justement de répondre à ce besoin en proposant un nouveau dispositif de support et de réglage en orientation d'organes divers par rapport à un plan de référence, dispositif simple, peu onéreux, facile à construire, ne nécessitant aucun entretien propre, léger et pouvant être de surcroît facilement nettoyé ou aseptisé.

Pour atteindre les buts ci-dessus, le dispositif de support est caractérisé en ce qu'il comprend

- une enveloppe en matière souple, non déformable élastiquement, délimitant une chambre étanche
- un raccord adapté sur l'enveloppe pour isoler ou mettre en relation la chambre avec le milieu environnant ou pour raccorder cette chambre à un circuit de mise sous vide relatif
- une masse de corps résilients occupant en partie le volume maximal de la chambre dans son état en relation avec le milieu environnant
- au moins deux platines solidaires de l'enveloppe et destinées à servir respectivement de pied de fixation amovible par rapport à un plan de référence et d'élément de support et de réglage en orientation pour un organe quelconque,
- et des organes d'ancrage solidaires des platines, dépourvus de liaison positive entre eux et dirigés vers le volume de la chambre pour plonger au moins en partie dans la masse de corps résilients.

Diverses autres caractéristiques ressortent de la description faite ci-dessous en référence aux dessins annexés qui montrent, à titre d'exemples non limitatifs, des formes de réalisation de l'objet de l'invention.

La fig. 1 est une coupe-élévation de l'objet de l'invention.

La fig. 2 est une coupe-élévation illustrant le dispositif dans une position caractéristique.

Les fig. 3 à 5 sont des coupes schématiques montrant différentes variantes de réalisation de certains des éléments constitutifs du dispositif.

Les fig. 6 et 7 sont des vues schématiques mettant en évidence une possibilité de construction du dispositif selon l'invention.

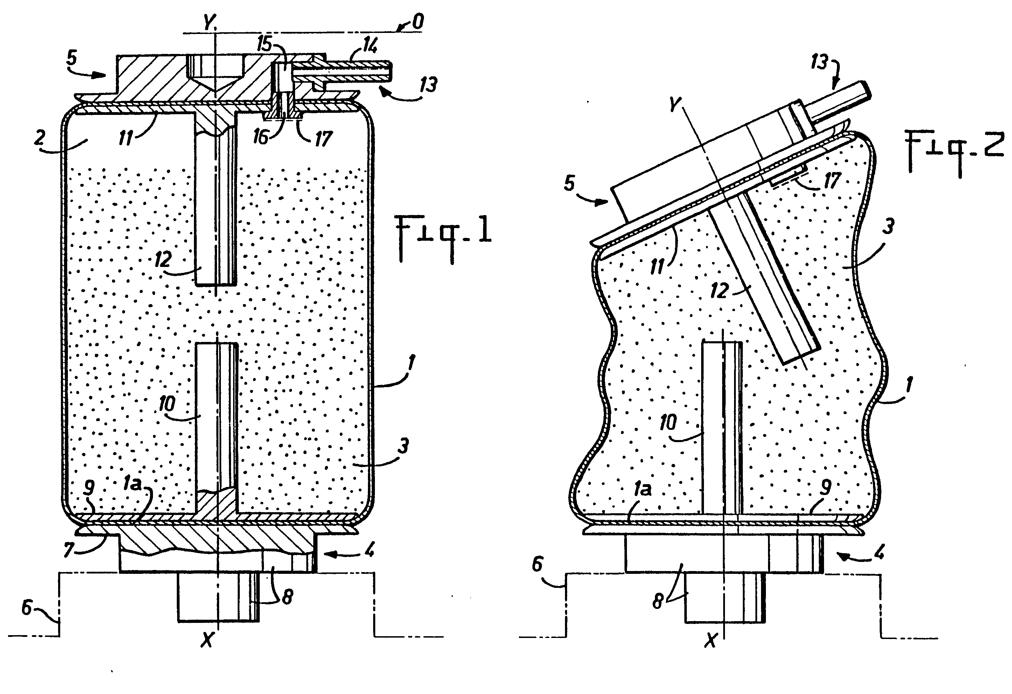

La fig. 1 montre le dispositif de support et de réglage en orientation comprenant, conformément à l'invention, une enveloppe 1 en matière souple non déformable élastiquement, réalisée de manière à délimiter une chambre 2 étanche. L'enveloppe 1 peut, par exemple, être constituée par une feuille de polyéthylène dont l'épaisseur est choisie en fonction de la résistance mécanique devant être conférée en fonction de l'application envisagée.

L'enveloppe 1 peut être réalisée de plusieurs façons convenables à partir d'une feuille ou encore d'une gaine tubulaire et, dans un tel cas, la délimitation de la chambre 2 intervient en rapportant par soudure toute partie complémentaire destinée à conférer à l'enveloppe 1 sa forme définitive.

Dans l'exemple illustré à la fig. 1, l'enveloppe 1 se présente sous une forme de manchon sensiblement cylindrique dont les faces transversales sont fermées. Il est bien évident que cette forme ne doit pas être considérée comme une caractéristique de l'invention. En effet, l'enveloppe 1 pourrait être réalisée sous la forme d'un coussin ou, encore, de plusieurs segments préorientés relativement, de sections droites transversales indifférentes.

La chambre 2 contient, intérieurement, une masse 3 de corps résilients occupant un volume compris entre 50 et 75 % du volume utile maximal de la chambre 2. Les corps résilients peuvent être constitués en de nombreuses matières et, par exemple, formés par des billes de polystyrène ayant un diamètre compris entre 0,8 et 1 mm. Les corps 3 peuvent aussi être constitués par des petits blocs de forme quelconque dont le volume peut varier de quelques mm³ à plusieurs dizaines de mm³.

L'enveloppe 1 est associée à deux platines 4 et 5 destinées à assumer respectivement une fonction de pied de fixation amovible par rapport à un plan de référence, tel que celui désigné par la référence 6, et une fonction d'élément de support et de réglage en orientation pour un organe quelconque. La platine de support 4 comprend une plaque 7 munie de moyens, tels que 8, permettant sa fixation, son adaptation ou son maintien dans les conditions requises par le domaine d'application sur le plan de référence 6. A cet égard, les moyens 8 peuvent donc être quelconques et ne sont donnés sur la fig. 1 qu'à titre non limitatif.

La platine 4 est, de préférence, associée à une contreplatine 9 avec laquelle elle enserre, maintient, serre ou pince une partie de l'enveloppe 1. Selon les dessins, la contreplatine 9 et la platine 4 apparaissent comme étant disposées de part et d'autre de l'épaisseur de paroi de l'enveloppe 1 sur laquelle ces pièces peuvent être, par exemple, fixées par collage. Il est évident que d'autres méthodes de fabrication peuvent être retenues et que, par exemple, la surface de l'enveloppe 1 peut comporter une ouverture ou plusieurs trous pour le passage d'organes complémentaires mâles et femelles présentés par la platine 4 et la contreplatine 9. Ces organes complémentaires peuvent être du type à serrage et coopérer alors avec des joints d'étanchéité ou avec une couche ou un film de matière adhésive, destiné à établir l'étanchéité aux interfaces en présence.

La contreplatine 9 est prolongée par un organe d'ancrage 10 orienté vers l'intérieur de la chambre 2 et destiné à être engagé ou à plonger au moins en partie dans la masse de corps résilients 3. Dans l'exemple illustré, l'organe d'ancrage 10 est formé par une broche dont la longueur est inférieure à la demi-longueur axiale de l'enveloppe 1.

Des moyens analogues ou identiques à ce qui est décrit ci-dessus sont mis en oeuvre pour constituer la platine 5 associée, selon l'exemple illustré, à une contreplatine 11 munie ou prolongée d'un corps d'ancrage 12 également formé par une broche cylindrique de longueur inférieure à la demi-longueur axiale de l'enveloppe 1.

Dans le cas présent, les organes d'ancrage 10 et 12 s'étendent selon l'axe des platines 4 et 5. Il pourrait, toutefois, être considéré que l'un au moins des organes d'ancrage se trouve décalé par rapport à un tel axe, schématisé en X et Y, pour ce qui concerne les platines 4 et 5. Dans tous les cas, les organes 10 et 12 sont dépourvus de liaison mutuelle positive.

L'enveloppe 1 est, par ailleurs, associée à un raccord 13 permettant de placer la chambre 2 en relation avec le milieu environnant ou permettant, également, d'isoler cette chambre par rapport à un tel milieu. La fonction du raccord 13 est aussi de pouvoir mettre la chambre 2 en relation avec une installation ou un circuit de mise sous vide relatif non représenté aux dessins.

Le raccord 13 est constitué par un embout 14, par exemple fixé dans un puits 15 ménagé dans la platine 5 pour communiquer par un trou 16 avec la chambre 2. Le trou 16 est équipé d'une grille 17 ou de tout autre moyen équivalent délimitant une ou plusieurs ouvertures de section de passage inférieure à la plus petite section des corps résilients 3. Le raccord 13 pourrait, bien entendu, être aussi adapté sur la platine 4 ou, encore, être porté directement par l'enveloppe 1.

Le dispositif décrit ci-dessus peut donc être adapté sur un plan de référence 6 pour porter, par la platine 5, un organe quelconque schématisé par le trait mixte 0 à la fig. 1.

Dans l'état représenté, l'enveloppe 1 ne possède aucune tenue propre susceptible de lui être conférée par la masse de corps 3, étant donné que cette dernière ne remplit que partiellement le volume de la chambre 2.

Lorsqu'il est souhaité maintenir l'organe 0 dans une orientation prédéterminée, il suffit de choisir manuellement cette orientation et de mettre en relation la chambre 2 avec un circuit de mise sous vide relatif approprié par l'intermédiaire du raccord 13. Le volume gazeux occupant la chambre 2 est extrait, ce qui a pour effet de réduire le volume de cette chambre, de plaquer l'enveloppe 1 sur et contre la masse de corps 3 qui se transforme, d'un état naturellement déformable, en une masse compacte résultant de l'arcboutement mutuel des corps 3 qui s'imbriquent relativement en étant maintenus serrés et compactés par l'intermédiaire de l'enveloppe 1.

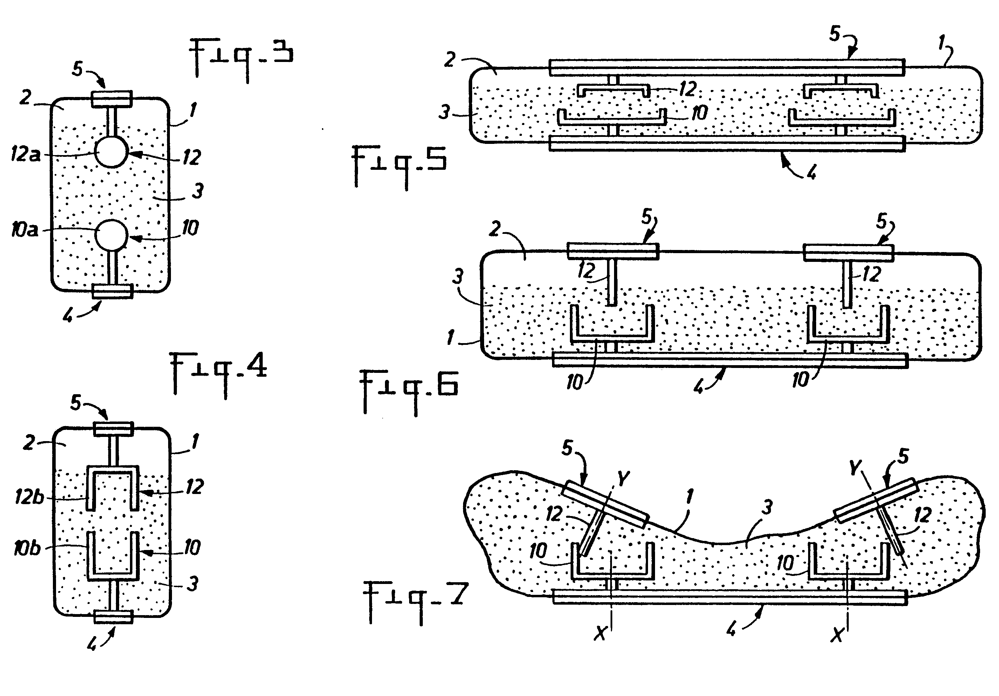

La masse de corps 3 et l'enveloppe immobilisent alors de façon ferme les organes d'ancrage 10 et 12 qui ont été placés relativement dans une position correspondant à l'orientation choisie pour l'organe 0, tel que cela est schématisé par la fig. 2.

Ainsi, l'organe d'ancrage 10 détermine une base sur laquelle est ancrée la masse 3 et l'enveloppe 1 dont la compacité assure l'immobilisation de l'organe d'ancrage 12 conférant l'orientation choisie à la platine 5.

Une rigidité maximale peut être obtenue en modifiant les caractéristiques du circuit de mise sous vide.

Lorsque l'immobilisation recherchée a été

obtenue, le raccord 13 est fermé pour isoler le dispositif du milieu environnant et du circuit de mise sous vide. De cette façon, l'enveloppe 1 et la masse 3 conservent l'état qui leur a été conféré et assurent ainsi le support et le maintien de l'organe 0 selon l'orientation choisie.

Il y a lieu de noter que l'immobilisation par rigidification de l'enveloppe 1 et de la masse 3 permet un réglage multidirectionnel et offre des possibilités de déplacement entre les axes X et Y ainsi qu'éventuellement un déplacement relatif entre les platines 4 et 5.

Par le dispositif de l'invention, il devient ainsi possible de réaliser rapidement, sans connaissance particulière et au moyen d'une simple manoeuvre d'un robinet d'isolement adapté sur le raccord 13, un support et une orientation réglables d'un organe devant être placé selon une direction précise choisie.

Lorsqu'il est souhaité modifier le réglage d'orientation, il suffit de replacer la chambre 2 en relation avec le milieu environnant pour admettre intérieurement la quantité de fluide gazeux nécessaire pour rompre l'arcboutement mutuel des corps 3 et libérer l'enveloppe 2 dont le caractère souple confère de nouveau alors au dispositif l'état initial permettant les réglages recherchés.

Les fig. 3 et 4 montrent, de façon schématique, que les organes d'ancrage 10 et 12 peuvent être constitués autrement que selon l'exemple illustré par la fig. 1 en comportant, par exemple, des sphères 10a ou 12a ou, encore, des fourchettes 10b ou 12b.

Le choix de la conformation des organes d'ancrage dépend du diamètre des corps résilients 3, de la matière de ces derniers et de la fermeté de support devant être recherchée.

Dans ce qui précède, il est fait référence à une enveloppe 1 en forme de manchon d'axe vertical. Il doit être compris qu'un tel manchon peut être supporté selon une direction différente et même qu'il est possible de placer le dispositif de façon suspendue à un support 6, de telle sorte que la platine 5 se trouve alors à la base du dispositif pour permettre la suspension d'un organe quelconque devant, néanmoins, être orienté et immobilisé selon une direction privilégiée.

La fig. 5 montre qu'il est possible, également, de réaliser l'enveloppe 1 sous la forme d'un coussin lorsqu'il est souhaité disposer d'un dispositif de faible encombrement vertical ou de faible épaisseur, apte à assurer le support et le réglage en orientation, d'organes de grande surface d'appui.

Dans un tel cas, il peut être prévu de faire comporter à chacune des platines 4 et 5 plusieurs organes d'ancrage 10 ou 12 de façon à établir une fixation répartie en fonction de la surface d'appui.

La fig. 6 montre qu'il est possible, également, de faire comporter à une enveloppe 1 plus de deux platines 5 indépendantes les unes des autres, de façon qu'il soit possible de conférer à chacune d'elles une orientation propre, comme cela ressort de la fig. 7.

Parmi les différentes applications possibles, l'une préférée consiste en la réalisation, au moyen du dispositif, d'un organe de support d'une aiguille de biopsie.

## Revendications

1. Dispositif de support et de réglage en orientation d'organes divers, par rapport à un plan de référence (6), caractérisé en ce qu'il comprend:
- une enveloppe (1) en matière souple, non déformable élastiquement, délimitant une chambre étanche (2),
- un raccord (13) adapté sur l'enveloppe pour isoler ou mettre en relation la chambre avec le milieu environnant ou pour raccorder cette chambre à un circuit de mise sous vide relatif,
- une masse (3) de corps résilients occupant en partie le volume maximal de la chambre dans son état en relation avec le milieu environnant,
- au moins deux platines (4) et (5) solidaires de l'enveloppe et destinées à servir, respectivement, de pied de fixation amovible par rapport à un plan de référence (6) et d'élément de support et de réglage en orientation pour un organe quelconque (0),
- et des organes d'ancrage (10 et 12) solidaires des platines, dépourvus de liaison positive entre eux et dirigés vers le volume de la chambre pour plonger, au moins en partie, dans la masse de corps résilients.

2. Dispositif selon la revendication 1, caractérisé en ce que chaque platine est associée à au moins un organe d'ancrage.

3. Dispositif selon la revendication 1 ou 2, caractérisé en ce que chaque platine (4, 5) est associée à une contreplatine (9 et 11) avec laquelle elle enserre l'enveloppe.

4. Dispositif selon la revendication 1, caractérisé en ce que le raccord (13) est monté sur la platine (5) de support et de réglage d'au moins un organe.

5. Dispositif selon la revendication 4, caractérisé en ce que le raccord (13) comporte une grille ou analogue (17) délimitant au moins une ouverture ayant une section de passage inférieure à la plus petite section des corps résilients.

6. Dispositif selon la revendication 1, caractérisé en ce que la masse (3) de corps résilients est au moins égale à 50 % du volume utile maximal de la chambre.

7. Dispositif selon la revendication 1, caractérisé en ce que l'enveloppe (1) est associée à une platine de fixation (4) et à au moins deux platines (5) de support et de réglage en orientation.

8. Dispositif selon la revendication 1, caractérisé en ce que les corps résilients sont constitués par des billes présentant un diamètre compris entre 0,8 et 1 millimètre.

9. Dispositif selon la revendication 1, caractérisé en ce que les corps (3) sont constitués par des blocs de forme quelconque ayant un volume compris entre quelques millimètres cubes à plusieurs dizaines de millimètres cubes.

**Patentansprüche**

1. Vorrichtung zum Tragen und zur verstellbaren Orientierung von verschiedenen Organen bezüglich einer Bezugsebene (6) gekennzeichnet durch
- eine Hülle (1) aus weichem elastisch nicht verformbaren Material, das eine dichte Kammer (2) umschließt,
- einen Verbinder (13), der auf der Hülle angepaßt ist, um die Hülle zu isolieren oder mit der Umgebung kommunizieren zu lassen oder um diese Kammer an ein Unterdrucksystem anzuschließen,
- eine Masse (3) aus elastischen Körpern, die teilweise das maximale Volumen der Kammer in dem Zustand, in dem die Kammer mit der Umgebung in Kontakt steht, ausfüllen,
- mindestens zwei mit der Hülle fest verbundene Platten (4 und 5), die dazu bestimmt sind jeweils als lösbare Befestigung bezüglich einer Bezugsebene (6) und als Trägerelement und als Element zur Einstellung der Orientierung für ein beliebiges Organ (0) zu dienen, und
- Verankerungsorgane (10 und 12) die fest mit den Platten verbunden sind und die nicht miteinander verbunden sind und in das Innere des Volumens der Kammer gerichtet sind um zumindest teilweise in die Masse aus aus elastischen Körpern einzutauchen.

2. Vorrichtung nach Anspruch 1, dadurch gekennzeichnet, daß jede Platte mit mindestens einem Verankerungsorgan versehen ist.

3. Vorrichtung nach Anspruch 1 oder 2, dadurch gekennzeichnet, daß jede Platte (4, 5) mit einer Konterplatte (9, 11) verbunden ist, mit der die Hülle eingeklemmt wird.

4. Vorrichtung nach Anspruch 1, dadurch gekennzeichnet, daß der Verbinder (13) auf der Platte zum Tragen und verstellbaren Orientieren mindestens eines Organs angeordnet ist.

5. Vorrichtung nach Anspruch 4, dadurch gekennzeichnet, daß der Verbinder (13) ein Gitter oder ein analoges Organ (17) aufweist, das mindestens eine Öffnung eingrenzt, die einen Durchlaßquerschnitt hat, der kleiner ist als der kleinste Querschnitt der elastischen Körper.

6. Vorrichtung nach Anspruch 1, dadurch gekennzeichnet, daß die Masse (3) der elastischen Körper mindestens 50 % des maximalen Nutzvolumens der Kammer einnimmt.

7. Vorrichtung nach Anspruch 1, dadurch gekennzeichnet, daß die Hülle (1) mit einer Befestigungsplatte (4) und mit mindestens zwei Platten (5) zum Tragen und Verstellen der Orientierung versehen ist.

8. Vorrichtung nach Anspruch 1, dadurch gekennzeichnet, daß die elastischen Körper aus Kugeln bestehen, die einen Durchmesser zwischen 0,8 und 1,0 mm aufweisen.

9. Vorrichtung nach Anspruch 1, dadurch gekennzeichnet, daß die Körper (3) aus beliebig geformten Blöcken bestehen, die jeweils ein Volumen haben, das zwischen mehreren Kubikmillimetern bis zu mehreren Zehnkubikmillimetern liegt.

**Claims**

1. Device for the directional support and adjustment of various members, with respect to a reference plane (6), characterized in that it comprises:
- an envelope (1) in elastically non-deformable supple material, defining a tight chamber (2),
- a connection piece (13) adapted on the envelope in order to isolate it from or connect it with the environment or to connect said chamber with a circuit for creating a relative vacuum,
- a mass (3) of resilient elements occupying part of the maximum volume of the chamber when the latter is connected with the environment,
- at least two plates (4) and (5) fast with the envelope and designed to act, respectively, as a removable fixing stand with respect to a reference plane (6) and as a directional support and adjustment element for a member of any type (0),
- and anchoring means (10 and 12) fast with the plates, having no positive link one with the other and being directed towards the volume of the chamber in such a way as to sink at least partly in the mass of resilient elements.

2. Device according to claim 1, characterized in that each plate is associated to at least one anchoring member.

3. Device according to claim 1 or 2, characterized in that each plate (4, 5) is associated to a counterplate (9 and 11) with which it grips the envelope.

4. Device according to claim 1, characterized in that the connection piece (13) is mounted on the plate (5) for supporting and adjusting at least one member.

5. Device according to claim 4, characterized in that the connection piece (13) comprises a grid or similar (17) defining at least one opening having a cross section less than the smallest section of the resilient elements.

6. Device according to claim 1, characterized in that the mass (3) of resilient elements is at least equal to 50 % of the maximum serviceable volume of the chamber.

7. Device according to claim 1, characterized in that the envelope (1) is associated to a fastening plate (4) and to at least two directional support and adjustment plates (5).

8. Device according to claim 1, characterized in that the resilient elements are constituted by beads having a diameter between 0.8 and 1 millimetre.

9. Device according to claim 1, characterized in that the elements (3) are constituted by blocks of any shape having a volume ranging between a few cubic millimeters and several tens cubic millimetres.

F̄ɪq.1

F̄ɪq.2

0 176 452

Fig.3

Fig.4

Fig.5

Fig.6

Fig.7

3